# EUROPÄISCHE PATENTSCHRIFT

(11) **EP 0 639 189 B1**
(45) Veröffentlichungstag und Bekanntmachung des Hinweises auf die Patenterteilung: **19.02.2003**
(21) Anmeldenummer: 92903347.0
(22) Anmeldetag: 23.01.1992
(51) Int. Cl.: C07D 401/04, C09K 19/40, C09K 19/34, C07D 405/14, C07F 7/08, G02F 1/13

(54) **PYRIDYLPYRIMIDINE, VERFAHREN ZU IHRER HERSTELLUNG UND IHRE VERWENDUNG IN FLÜSSIGKRISTALLINEN MISCHUNGEN**
PYRIDYLPYRIMIDINES, A METHOD OF PREPARING THEM, AND THEIR USE IN LIQUID-CRYSTAL MIXTURES
PYRIDYLPYRIMIDINES, PROCEDE DE PREPARATION ET APPLICATION DANS DES MELANGES A CRISTAUX LIQUIDES

(30) Priorität: 24.01.1991 DE 4102016
(43) Veröffentlichungstag der Anmeldung: 22.02.1995
(73) Patentinhaber: Aventis Research & Technologies GmbH & Co. KG, 65926 Frankfurt am Main (DE)
(72) Erfinder: ESCHER, Claus, D-6109 Mühltal (DE); Illian, Gerhard, D-6000 Frankfurt am Main 71 (DE); HEMMERLING, Wolfgang, D-6231 Sulzbach/Taunus (DE); WINGEN, Rainer, D-6234 Hattersheim am Main 1 (DE)
(74) Vertreter: Bardehle, Heinz, Dipl.-Ing.
(86) Internationale Anmeldenummer: EP9200142
(87) Internationale Veröffentlichungsnummer: WO92012974

(56) Entgegenhaltungen:
- EP-A- 0 160 790
- US-A- 4 668 425

## Beschreibung

Die ungewöhnliche Kombination von anisotropem und fluidem Verhalten der Flüssigkristalle hat zu ihrer Verwendung in einer Vielzahl von elektro-optischen Schaft- und Anzeigevorrichtungen geführt. Dabei können ihre elektrischen, magnetischen, elastischen und/oder thermischen Eigenschaften zu Orientierungsveränderungen genutzt werden. Optische Effekte lassen sich dann beispielsweise mit Hilfe von Doppelbrechung, der Einlagerung dichroitisch absorbierender Farbstoffmoleküle ("guest host mode") oder der Lichtstreuung erzielen.
Zur Erfüllung der ständig steigenden Praxisanforderungen auf den verschiedenen Anwendungsgebieten besteht laufend ein Bedarf an neuen verbesserten Flüssigkristall ("liquid crystal")-Mischungen und somit auch an einer Vielzahl mesogener Verbindungen unterschiedlicher Struktur. Dies gilt sowohl für die Gebiete, bei denen nematische LC-Phasen (z.B. TN = "twisted nematic", STN = "supertwisted nematic", SBE = "supertwisted birefringence effect", ECB = "electrically controlled birefringence") verwendet werden, als auch für solche mit smektischen LC-Phasen (z.B. ferroelektrische, elektrokline).
Viele der für LC-Mischungen geeigneten Verbindungen lassen sich durch ein Aufbauprinzip beschreiben (siehe z.B. J. Am. Chem. Soc. 108, 4736 (1986), Struktur I; Science 231, 350 (1986, Fig. 1 A; J. Am. Chem. Soc. 108, 5210 (1986), Fig. 3), bei dem Kerne aus cyclischen Verbindungen - Aromaten, Heteroaromaten. aber auch gesättigte Ringsysteme - mit geradkettigen oder in der Kette ourch kleine Gruppen (z.B. Methyl, Chlor) substituierten und somit verzweigten Alkylseitenketten verknüpft sind.

Die Verwendung von Pyrimidin-Verbindungen als Komponenten von Flüssigkristallmischungen ist z.B. aus US 4713197, US 4725688, US 4752414, US 4765924, US 4776977, US 4804759, US 4812258, US 4820839, US 4892679, US 4906401 und US 4906752 bekannt.
Ähnliche Pyridin-Verbindungen, ebenfalls für den Einsatz in Flüssigkristallmischungen, sind z.B. aus EP-B 242716, EP-B 240320, EP-A 391203, US 4765924 und US 4952335 bekannt.
Flüssigkristallmischungen, die Pyridylpyrimidine der Formel (A) enthalten, werden in JP-A 61280489 beschrieben als Verbindungen mit nematischen Mesophasen bzw. günstigem Einfluß auf nematische Flüssigkristallmischungen. 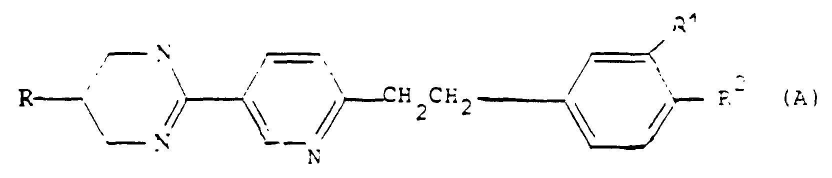 (R: C₁₋₁₀ alkyl oder alkyloxy; R¹: H,Cl,F; R²: F,Cl,CN, C₁₋₁₀ alkyl oder alkyloxy)

Pyrimidinyl-pyrimidine der Formel (B) wurden von H. Zaschke et al. (7th Liquid Crystal Conference of Socialist Countries, 1987, Parduvice, CSFR; E-8) vorgestellt.

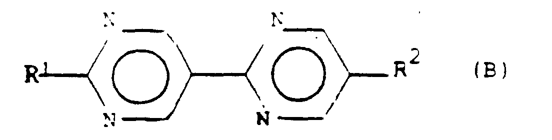

Zweikernige Vertreter weisen keine oder nur eine monotrope smektische Phase auf, während dreikernige Verbindungen S_{E}- und/oder S_{A}-Phasen aufweisen.

Aufgabe der vorliegenden Erfindung ist es, neue mesogene Verbindungen zur Verfügung zu stellen, die mit anderen Komponenten zu LC-Mischungen mit vorteilhaften Eigenschaften kombiniert werden können. Die nachstehend definierten Verbindungen lösen diese Aufgabe:

Flüssigkristalline Pyridylpyrimidin-Verbindungen der Formel (I) 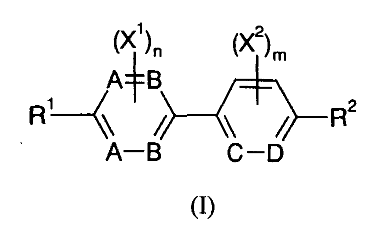 in der bedeuten:
A gleich N und B gleich CH oder A gleich CH und B gleich N
C gleich N und D gleich CH oder C gleich CH und D gleich N
R¹ und R² gleich oder verschieden lineares oder verzweigtes Alkyl mit 1 bis 16 C-Atomen, Alkyloxy oder Alkyldimethylsilylalkyl(oxy) der Formel R⁷-Si(CH₃)₂-G oder einer der nachfolgenden Reste 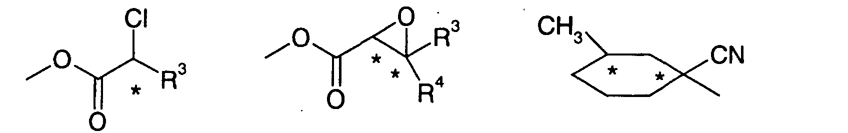 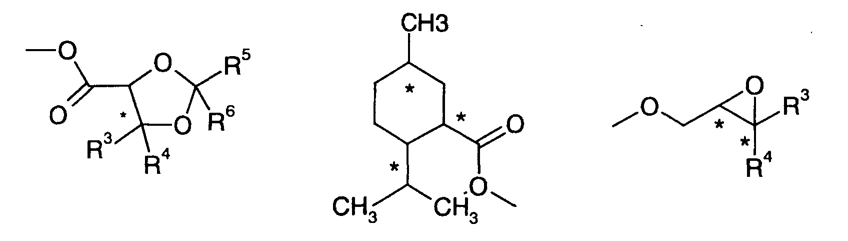 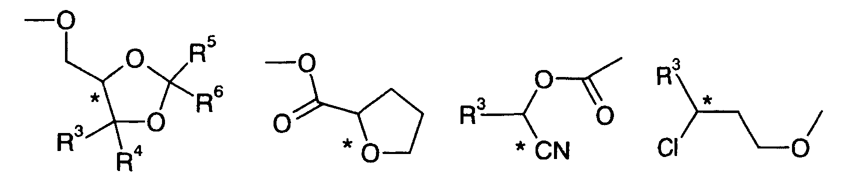 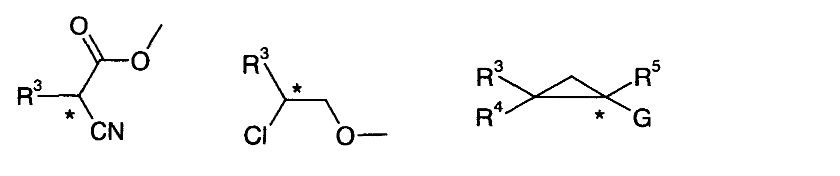 oder trans-Alkylcyclohexylcarbonyloxy,
X¹, X² gleich F, Cl, CN, vorzugsweise F,
n, m gleich oder verschieden 0, 1 oder 2, vorzugsweise 0,
R³, R⁴, R⁵, R⁶ H, geradkettiges oder verzweigtes Alkyl mit 1 bis 16 C-Atomen oder Alkenyl mit 2 bis 16 C-Atomen, bei denen auch eine nicht der Verknüpfungsstelle benachbarte -CH₂-Gruppe durch -O-, -CO-O- oder -O-COersetzt sein können, oder
R³ und R⁴ bzw. R⁵ und R⁶ zusammen cyclisches Alkyl mit 3 bis 8 C-Atomen R⁷ geradkettiges oder verzweigtes Alkyl mit 1 bis 16 C-Atomen oder Alkenyl mit 2 bis 16 C-Atomen, bei denen auch eine oder zwei nicht benachbarte -CH₂-Gruppen durch -O-, -CO-O- oder -O-CO- ersetzt sein können, mit der Maßgabe, daß Silizium nur an ein C gebunden ist, das H und/oder C als Nachbaratome hat, oder cyclisches Alkyl mit 3 bis 8 C-Atomen,
G geradkettiges oder verzweigtes Alkylen mit 1 bis 16 C-Atomen oder Alkenylen mit 2 bis 16 C-Atomen, bei denen eine oder zwei nicht benachbarte -CH₂-Gruppen durch -O-, -S-, -O-CO-, -CO-O-, -S-CO- oder -CO-S- ersetzt sein können, mit der Maßgabe, daß Si nur an ein C-Atom gebunden ist, das H und/oder C-Atome als Nachbaratome hat.

Bevorzugt sind Verbindungen, bei denen einer der beiden Reste R¹ oder R² trans-Alkylcyclohexylcarbonyloxy bedeutet.

Besonders bevorzugt sind Pyridylpyrimidin-Verbindungen der Formeln (II) und (III) und (IIIa) 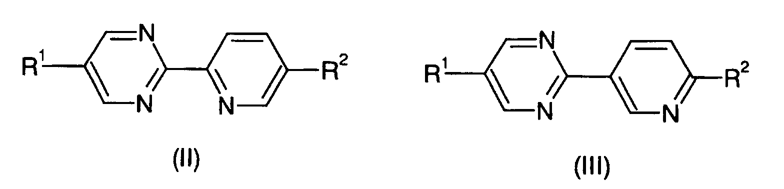 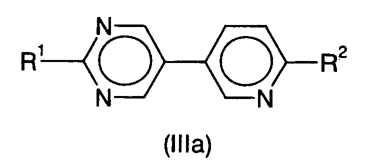 wobei R¹ und R² die oben genannte Bedeutung haben.

Die erfindungsgemäßen Verbindungen zeichnen sich insbesondere dadurch aus, daß sie breite smektische C-Phasenbereiche und niedrige Schmelzpunkte aufweisen.
Zudem besitzen die erfindungsgemäßen Verbindungen meist neben den ergänzend smektischen C-Phasen auch smektische A-Phasen, welche man in praxisgerechten Mischungen für gute Orientierungen benötigt.
Sie können nach an sich bekannten Standardreaktionen erzeugt werden, wobei im allgemeinen ein reaktionsfähiges Pyridinderivat mit einer bifunktionellen, elektrophilen Verbindung unter Bildung eines Pyrimidinsystems zur Reaktion (Kondensation) gebracht wird, z.B. auf dem im Schema I dargestellten Weg: 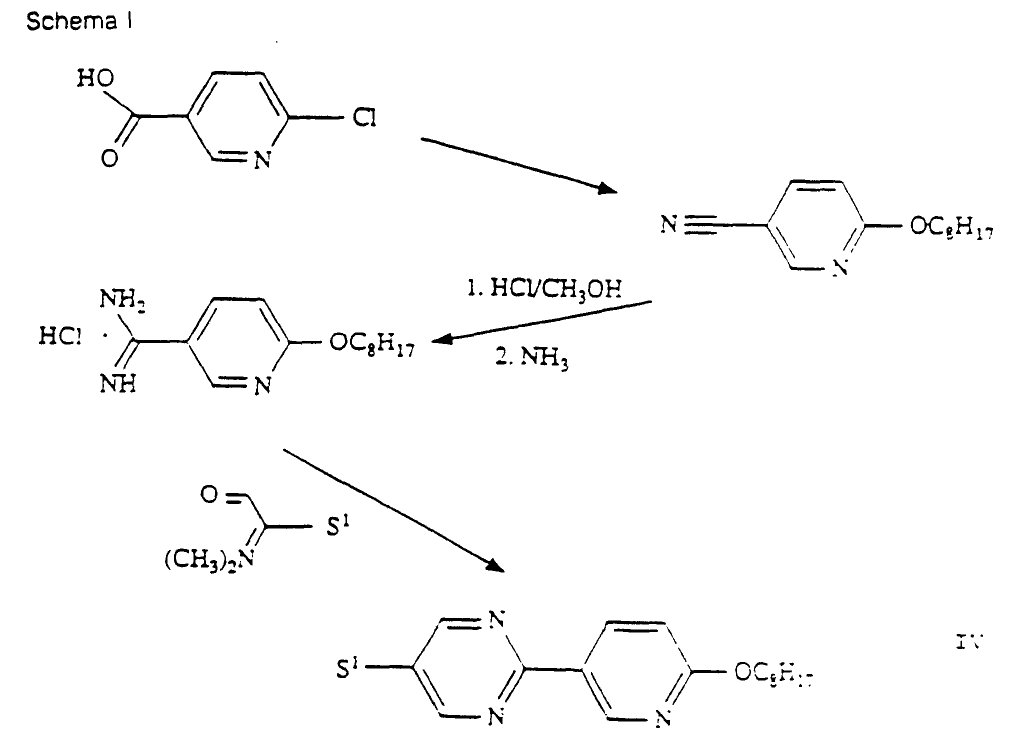 6-Chlornicotinsäure wird in mehreren Stufen zu 6-Octyloxy-nicotinsäurenitril umgesetzt. Die Amidin-Bildung auf dem üblichen Weg ergibt 6-Octyloxynicotinamidin Hydrochlorid. Kondensation mit einem Dimethylaminoacrolein-Derivat führt zum Pyridylpyrimidin (IV), wobei
S¹ gleich R¹ ist oder einer Schutzgruppe wie Benzyloxy, Trialkylsilyoxy, Tetrahydropyranyloxy, Acyloxy, Benzylthio oder einer anderen, in "T.W.Greene, Protective Groups in Organic Systems. Wiley (1981) aufgeführten, geeigneten Schutzgruppe oder einer Hydroxy-Gruppe entspricht. Die Abspaltung der Schutzgruppen kann nach den in derselben Literaturstelle empfohlenen Methoden erfolgen.
Pyridylpyrimidine der Formel (V) mit S² gleich oder verschieden S¹ können ausgehend von z.B. 5-Hydroxy-2-methylpyridin erhalten werden, das zunächst zur Carbonsäure oxidiert wird. Die weitere Synthese über Nitril und Amidin erfolgt nach den üblichen Methoden [(z.B. H. Foerster and J. Walker, J.Chem.Soc. 1948, 1939 und H. Zaschke und H. Schubert, J. Prakt. Chem. 312, 494 (1970) und ibid. 315, 113 (1973)].

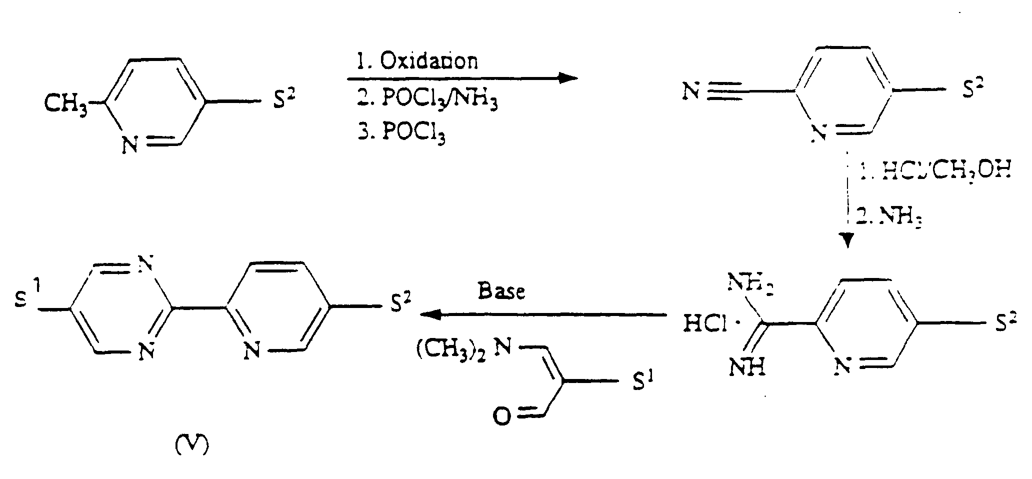

Verbindungen der Formel (VI) mit X' gleich F oder Cl können auf folgende Weise sythetisiert werden, 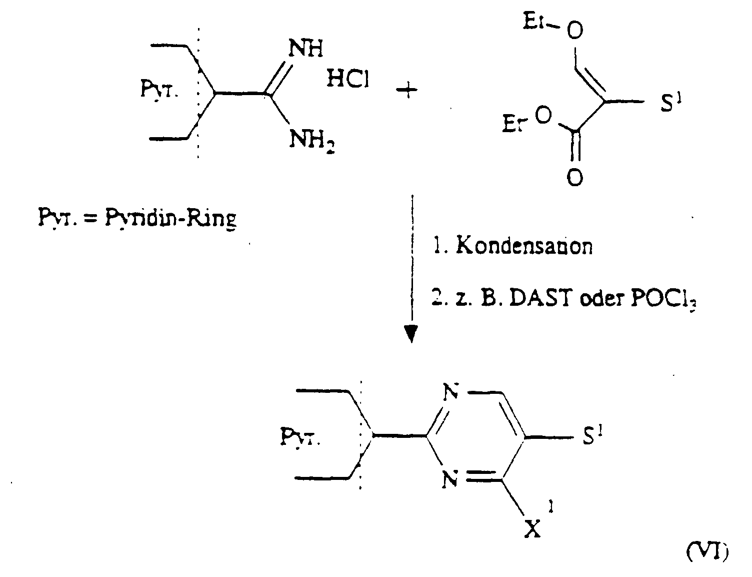 indem z.B. nach A. Boller et al. Z. Naturforsch. 33b, 433-438 (1978) ein Pyridylamidin mit einem 2-Ethoxymethylencarbonsäureester-Derivat kondensiert wird. Die 4-Hydroxy-Gruppe am Pyrimidin-Ring kann dann mit POCl₃ bzw. Diethylamino-schwefeltrifluorid (DAST) gegen Chlor bzw. Fluor ausgetauscht werden.

Um zu X¹ gleich CN zu gelangen, setzt man das Hydroxy-Zwischenprodukt mit X¹ gleich Br, das in Analogie zur Chlor-Verbindung erzeugt wird, mit Kupfercyanid in einem geeigneten Lösungsmittel wie N-Methyl-pyrrolidon um.
Ether der allgemeinen Formel (I) können, sofern die Etherstruktur nicht bereits schon vor der Heterocyclen-Bildung vorhanden war, hergestellt werden aus Phenolen der Formeln (IV), (V) oder (VI) mit S¹ oder S² gleich OH oder O-Alkali durch Reaktion mit monofunktionell reaktionsfähigen Alkyl- oder Alkenylhalogeniden, oder aus monofunktionell reaktionsfähigen Alkoholen - wie sie sich aus den unter R¹ aufgeführten Resten ableiten lassen - bzw. deren Mesylaten oder Tosylaten, wobei die Synthese dieser Reaktionskomponenten als bekannt vorausgesetzt werden kann.

So können beispielsweise Phenole oder Thiophenole der Formeln (IV), (V) oder (VI) mit Hydroxy-Verbindungen in Anwesenheit von Triphenylphosphin/Azodicarbonsäurediester (Mitsunobu-Reaktion, z.B. in J. Chem. Soc. Perkin Trans 1 1975, 461) verknüpft werden. Es können auch die separat oder intermediäte erzeugten Alkali- oder Erdalkalisalze dieser mesogenen Hydroxy- oder MercaptoVerbindungen mit Halogen-, Toluolsulfonyloxy- oder Methylsulfonyloxy-Verbindungen umgesetzt werden (Williamson-Reaktion, z.B. in Patai, The Chemistry of the Ether Linkage, Interscience Publishers, New York 1967, S. 446-468). Phenole oder Thiophenole der Formeln (II), (III) oder (IV) können auch mit Carbonsäuren, wie sie sich aus R¹ ableiten lassen, unter Kondensationsbedingungen (z.B. March, Advanced Organic Chemistry, 3rd Ed., Wiley-interscience, New York 1985, S. 240, 348-353) oder unter den Bedingungen der Williamson-Reaktion zu Estern oder Thioestern umgesetzt werden. In gleicher Weise ist dies auch mit mesogenen Carbonsäuren (S¹/S² gleich COOH) und den aus R¹ abgeleiteten Hydroxy-Verbindungen möglich.

Die Verbindungen der Formel (I) können auch erhalten werden, indem z.B. 2-Alkyl- bzw. 2-Alkoxy-pyridin-5-yl-boronsäuren - hergestellt werden, z.B. nach "Houben-Weyl, Methoden der Organischen Chemie, Bd. 13/3a, S. 635-647" oder "Basil J. Wakefield, The Chemistry of Organolithium Compounds, Pergamon Press. 1974 - mit 2- oder 5-Brom-substituierten Pyrimidinderivaten metallorganisch gekuppelt werden, z.B. nach Tetrahedron 28, 5093 (1987), oder Mol. Cryst. Liq. Cryst. 204, 91 (1991) oder J. Chem. Soc. Perkin Trans. II 1989, 2041.

Denkbar ist nach der prinzipiell gleichen Methodik auch die Kupplung von Pyrimidinboronsäuren mit Brom-pyridin-Verbindungen.

Die Erfindung betrifft ferner eine flüssigkristalline, insbesondere eine ferroelekirische Mischung bestehend aus 2 bis 20, vorzugsweise 2 bis 15 Komponenten, wobei die Mischung mindestens eine Verbindung der Formel (II) enthält. Die anderen Bestandteile werden vorzugsweise ausgewählt aus den bekannten Verbindungen mit nematischen, cholesterischen und/oder geneigt-smektischen Phasen, dazu gehören beispielsweise Schiffsche Basen, Biphenyle, Terphenyle, Phenylcyclohexane, Cyclohexylbiphenyle, Pyrimidine, Zimtsäureester, Cholesterinester und terminat-polare mehrkernige Ester von p-Alkylbenzoesäuren.

Von der oder den erfindungsgemäßen Verbindung(en) enthalten die Flüssigkristallmischungen im allgemeinen 0,1 bis 70 Mol-%, bevorzugt 0,5 bis 50 Mol.-%, insbesondere 1 bis 40 Mol.-%.

Darüber hinaus betrifft die Erfindung eine elektrooptische Schalt- und Anzeigevorrichtung (Display) enthaltend Trägerplatten, ein Flüssigkristall-Medium, Elektroden, mindestens eine Orientierungsschicht sowie gegebenenfalls Polarisatoren und weitere Hilfsschichten, die als Flüssigkristall-Medium eine erfindungsgemäße Mischung wie oben beschrieben enthält.

Für die ferroelektrischen Flüssigkristallmischungen wurden die Werte für die spontane Polarisation Pₛ[nC/cm²], die optische Schaltzeit τ[µS], und den effektiven Schaltwinkel ⊖_{eff}[°] wie folgt bestimmt:
Die P_{S}-Werte werden nach der Methode von H. Diamant et al. [Rev. Sci. Instr. 28, 30 (1957)] gemessen, wobei Meßzellen mit 2 µm Elektrodenabstand und geriebenem Polyimid als Orientierungsschicht verwendet werden.
Zur Bestimmung von τ und ⊖_{eff} wird die Meßzelle auf dem Drehtisch eines Polarisationsmikroskops zwischen gekreuztem Analysator und Polarisator befestigt. Durch Drehen des Tisches wird die Position des Tisches mit minimalem Lichtdurchgang für die beiden Schaltzustände in der Zelle bestimmt. Die Differenz der beiden Positionen am Drehtisch ist gleich dem doppelten effektiven Tiltwinkel. Mit Hilfe einer Photodiode erfolgt die Bestimmung der Schaltzeit τ, indem die Anstiegszeit des Lichtsignals von 10 auf 90% Signalhöhe gemessen wird. Die Schaltspannung besteht aus Rechteckpulsen und beträgt ± 10/µm.
Die Phasenumwandlungstemperaturen werden bei Aufheizen mit Hilfe eines Polarisationsmikroskops anhand der Texturänderungen bestimmt. Die Bestimmung des Schmelzpunkts wurde hingegen mit einem DSC-Gerät durchgeführt. Die Angabe der Phasenumwandlungstemperaturen zwischen den Phasen
Nematisch (N bzw. N*)
Smektisch-C (S_{C} bzw. S_{C}*)
Smektisch-A (S_{A} bzw. S_{A}*)
Kristallin (X bzw. K)
erfolgt in °C und die Werte stehen zwischen den Phasenbezeichnungen in der Phasenfolge.

### Beispiel 1

### Synthese von 2-(6-Octyloxypyrid-3-yl)-5-octyloxypyrimidin.

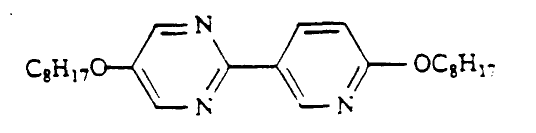

12 g (400 mmol) 80% NaH in 30 ml trockenem Dioxan werden bei 70°C tropfenweise mit 52 g (400 mmol) Octanol in 150 ml Dioxan versetzt. Nach 2stündigem Erhitzen bei Rückfluß tropft man 55 g (300 mmol) 6-Chlornicotinsäureethylester in 1150 ml Dioxan zu und kocht die Reaktionslösung weitere 5 Stunden am Rückfluß. Man gießt die Lösung auf 400 ml Eiswasser, trennt die Phasen und extrahiert die wässrige Phase mehrfach mit Ether. Die vereinigten organischen Phasen werden mit gesättigter Kochsalzlösung gewaschen, über Magnesiumsulfat getrocknet und im Vakuum eingeengt. Das Produkt wird chromatographisch an Kieselgel gereinigt.
55,8 g 6-Octyloxynicotinsäureester werden unter Rühren zu 500 ml konzentrierter Ammoniak-Lösung gegeben. Man läßt über Nacht stehen. Der Niederschlag wird abgesaugt und mit wenig Wasser gewaschen. Die Mutterlauge wird weitgehend eingeengt, wobei weiteres Produkt ausfällt. Es wird aus Wasser umkristallisiert.

45 g (180 mmol) 6-Octyloxynicotinamid und 41,4 g (270 mmol) Phosphoroxychloric werden 2 Stunden auf 125 bis 130°C erwärmt. Man engt im Vakuum ein und überschichtet den Rückstand mit 250 ml Chloroform. Unter heftigem Rühren werden 450 ml konzentrierte Sodalösung zugegeben. Nach 10minütigem Rühren trennt man die Phasen und extrahiert die wässrige mehrmals mit Chloroform. Die vereinigten organischen Phasen werden mit Wasser gewaschen, über Magnesiumsulfat getrocknet und im Vakuum eingeengt. Man reinigt das Rohprodukt durch Chromatographie an Kieselgel.
21,8 g (100 mmol) 6-Octyloxynicotinsäurenitril und 6,42 g (200 mmol) absolutes Methanol werden in 200 ml Dioxan vorgelegt. Bei 0°C wird Chlorwasserstoff bis zur Sättigung eingeleitet. Man läßt eine Nacht bei 0°C stehen und filtriert unter Schutzgas ab. Der Niederschlag wird im Vakuum über KOH bei 50°C getrocknet. 30,1 g (100 mmol) 6-Octyloxynicotinsäureiminomethylester, aufgeschlemmt in 300 ml EtOH, gibt man zu 110 ml bei 20°C mit Ammoniak gesättigtem Ethanol. Man rührt 2,5 Stunden bei 60°C und über Nacht bei Raumtemperatur. Die Reaktionslösung wird bis fast zur Trockene eingeengt und mit Ether ausgerührt. Der Niederschlag wird abgesaugt und im Vakuum getrocknet.
0,92 g (40 mmol) Na werden in 30 ml Ethanol gelöst. Man tropft 5,46 g (24 mmol) 2-Octyloxy-3-dimethylaminoacrolein in 10 ml Ethanol bei Raumtemperatur zu und gibt portionsweise 5,7 g (20 mmol) Amidinhydrochlorid der 6-Octyloxynicotinsäure zu. Das Gemisch wird 20 Stunden am Rückfluß erhitzt. Man stellt danach auf pH 3, engt ein, nimmt in 50 ml Wasser auf und extrahiert mit Dichlormethan. Die organische Phase wird über Magnesiumsulfat getrocknet und im Vakuum eingeengt. Man chromatographiert die Endverbindung an Kieselgel mit Dichlormethan und kristallisiert aus Hexan um. Das Produkt hat folgende Phasenfolge: X 58 S_{C}60S_{A}95,3 I.

### Beispiel 2

### Synthese von (2S,3S)-3-Butyloxiran-2-methyl-[2-(6-octyloxypyrid-3-yl)pyrimidin-5-yl]-ether

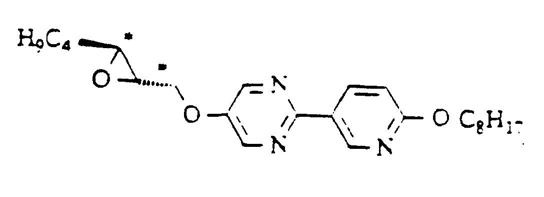

Die Herstellung des Octyloxypyridinamidin-Hydrochlorids und die Kondensation mit 2-Benzyloxy-3-dimethylaminoacrolein erfolgt in Analogie zu Beispiel 1. 1,96 g (5 mmol) 5-Benzyloxy-2-(6-octyloxypyrid-3-yl)-pyrimidin werden in 40 ml THF in Gegenwart einer Spatelspitze para- Toluolsulfonsäure und 200 mg 10% Palladium (auf Aktivkohle) hydriert. Man filtriert über Coriolite, engt im Vakuum ein, nimmt in 50 ml Dichlormethan auf und wäscht mit 20 ml Wasser. Die organische Phase wird über Magnesiumsutfat getrocknet und im Vakuum eingeengt. Man kristallisiert aus Petrolether um. 1,36 g (4,5 mmol) 5-Hydroxy-2-(6-octyloxypyrid-3-yl)-pyrimidin in 10 ml DMF werden zu 240 mg (8 mmol) 80% NaH in 5 ml DMF getropft. Anschließend fügt man 1,03 g (5 mmol) (2S,3S)-3-Butyloxiran-2-methylmethansulfonsäureester in 10 ml DMF hinzu und rührt 1 Stunde bei 60°C. Die Reaktionslösung wird in 50 ml gesättigter Citronensäure-Lösung hydrolysiert. Die organische Phase wird abgetrennt und die wässrige dreimal mit Dichlormethan extrahiert. Man wäscht die vereinigten organischen Phasen mit Wasser, trocknet sie über Magnesiumsulfat und engt sie im Vakuum ein. Das Rohprodukt wird an Kieselgel mit Dichlormethan chromatographiert und aus Heptan umkristalliert. 1,1 g (60% Ausbeute). Das Reinprodukt hat die folgende Phasenfolge:
X 72 (385_{C}* 42) S_{A}* 105 I.
[α]_{D} = -16,6° (c=1.7, CHCl₃).

### Beispiel 3

### Synthese von trans-4-Pentylcyclohexancarbonsäure-[2-(6-octyloxypyridin-3-yl)-pyrimidin-5-yl]-ester

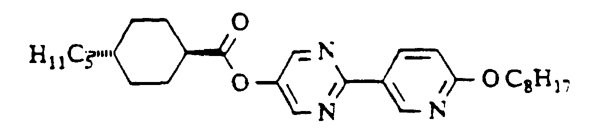

0,21 g (1 mmol) Dicycloheanylcarbodiimid in 4 ml Dichlormethan werden mit 0.20 g (1 mmol) trans-4-Pentylcyclohexancarbonsäure, 0,30 g (1 mmol)
5-Hydroxy-2-(6-octyloxypyridin-3-yl)-pyrimidin (Herstellung siehe Beispiel 2) sowie 0,01 g (0,1 mmol) 4-Dimethylaminopyridin versetzt und eine Nacht bei Raumtemperatur gerührt. Man filtriert, wäscht den Rückstand mit wenig Dichlormethan nach und engt das Filtrat ein. Nach Chromatographie an Kieselgel mit Hexan/Ethylacetat 5:1 sowie erneuter Chromatographie an Kieselgel mit Dichlormethan und Kristallisation aus Hexan erhält man 0.22 g (73% Ausbeute) des gewünschten Produkts mit der Phasenfolge: X 112 S_{A} 195 I.

### Beispiel 4

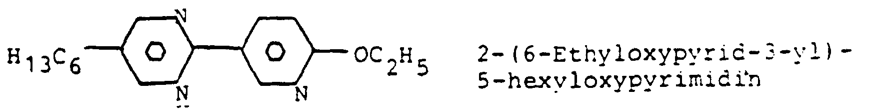

Die Herstellung des Ethyloxypyridinamidin-Hydrochlorids und die Kondensation mit 2-Benzyloxy-3-dimethylaminoacrolein erfolgt in Analogie zu Beispiel 1. 0,22 g (1 mmol) 5-Hydroxy-2-(6-ethyloxypyrid-3-yl)-pyrimidin in 3 ml DMF lösen, zu 60 mg (2 mmol) 80% NaH in 2 ml DMF getropft. Anschließend 0.22 g (1.33 mmol) Bromhexan in 4 ml DMF zutropfen und 2 Stunden bei 60°C rühren. Die Reaktionslösung in 10 ml H₂O gießen, 3mal mit Dichlormethan extrahieren. organische Phasen mit gesättigter NaCI-Lösung und H₂O waschen, mit MgSO₄ trocknen und einengen. Das Rohprodukt wird an S₁O₂ mit Hexan/Ethylacetat = 5/1 chromatographiert und aus Hexan umkristallisiert.
X 69 S_{A} 95 I

### Beispiel 5

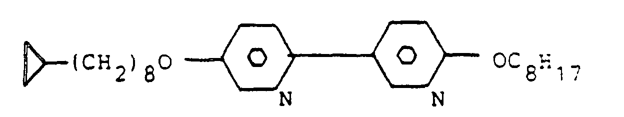

Analog Beispiel 2, Umsetzung von 5-Hydroxy-2-(6-octyloxypyrid-3-yl)-pyrimidin mit 8-Cyclopropylbromoctan zu
8-Cyclopropyloctyl-[2-(6-octyloxypyrid-3-yl)-pyrimidin-5-yl]-etner
X 78 S_{C} S_{A} 85 I

### Beispiel 6

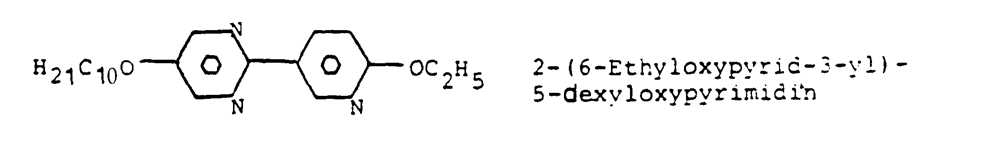

Analog Beispiel 5 aus 5-Hydroxy-2-(6-ethyloxypyrid-3-yl)-pynmioin und Bromdecan
X 54 S_{A} 100 I

### Beispiel 7

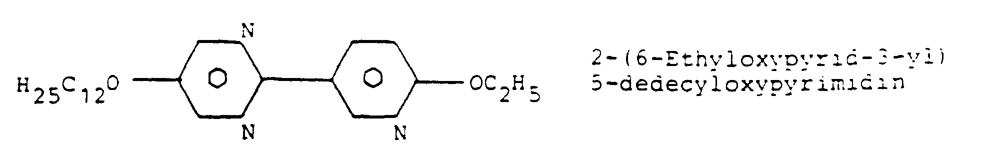

Analog Beispiel 5 aus 5-Hydroxy-2-(6-ethyloxypyrimid-3-yl)-pyrimidin und Bromdodecan
X 57 S_{A} 98 I

### Beispiel 8

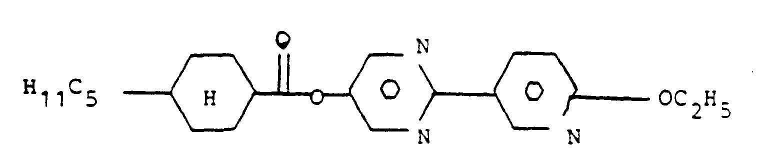

trans-4-Pentylcyclohexancarbonsäure -[2-(6-ehtyloxypyridin-3-yl)-pyrimidin-5-yl]-ester
Analog Beispiel 3 aus 5-Hydroxy-2-(6-ethyloxypyridin-3-yl)-pyrimidin und trans-4-Pentylcyclohexancarbonsäure
X 128 S_{A} 199 N 210 I

### Beispiel 9

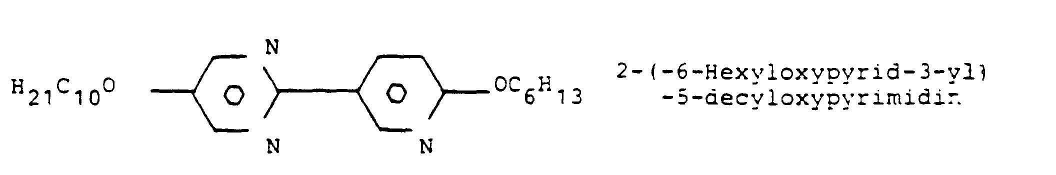

Die Herstellung des 6-Hexyloxypyridin-3-amidinhydrochlorids und die Kondensation mit 2-Benzyloxy-3-dimethylaminoacrolein erfolgt in Analogie zu Beispiel 1, die Alkylierung von 5-Hydroxy-2-(6-hexyloxypyridin-3-yl)-pyrimidin analog Beispiel 5.
Es wird zuletzt aus Heptan umkristallisiert.
X 54 S_{C} 82 S_{A} 97 I

### Beispiel 10

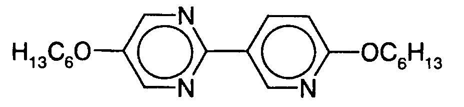

2-(6-Hexyloxypyrid-3-yl)-5-Hexyloxypyrimidin
Analog Beispiel 9 aus 5-Hydroxy-2-(6-hexyloxypyridin-3-yl)-pyrimidin und Bromhexan.
X 48 S_{A} 95 I

### Beispiel 11

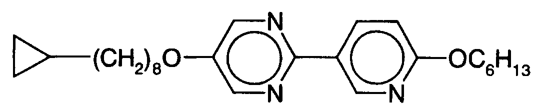

2-(6-Hexyloxypyrid-3-yl)-5-(8-cyclopropyloctyloxy)-pyrimidin
Analog Beispiel 9 aus 5-Hydroxy-2-(6-hexyloxypyridin-3-yl)-pyrimidin und 8-Cyclopropylbromoctan
X 58 S_{C} 76 S_{A} 85 I

### Beispiel 12

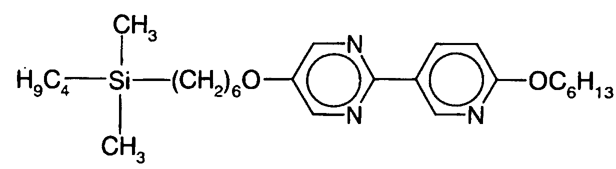

2-(6-Hexyloxypyrid-3-yl)-S-(7,7-dimethyl-7-sila undecyloxy)-pyrimidin Analog Beispiel 9 aus 5-Hydroxy-2-(6-hexyloxypyridin-3-yl)-pyrimidin und 7,7-Dimethyl-7-silabromundecan
X 24 S_{C} 28 I N.N.

### Beispiel 13

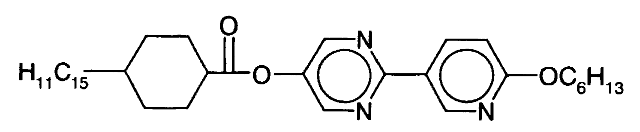

trans-4-Pentylcyclohexylcarbonsäure-[2-(6-hexyloxypyridin-3-yl)-pyrimidin-Syl)-ester]
Analog Beispiel 8 aus 5-Hydroxy-2-(6-hexyloxypyridin-3-yl)-pyrimidin und trans-4-Pentylcyclohexancarbonsäure
X 114 S_{A} 201 I

### Beispiel 14

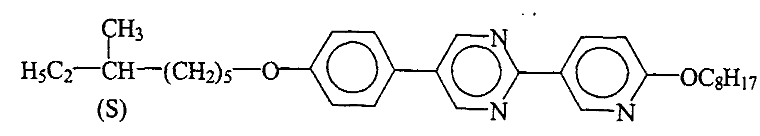

(S)-2-(6-Octyloxypyridin-3-yl)-5[4-(6-methyloctyloxy)-phenyl]-pyrimidin Analog Beispiel 1 aus 6-Octyloxynicotinsäureamidinhydrochlorid und (S)-2-[4-(6-methyloctyloxy)phenyl]-1-dimethylamino-3-dimethylaminopropanoperchlorat.
X 76 S_{C}^{*} 171 S_{A}^{*} 180 I

### Beispiel 15

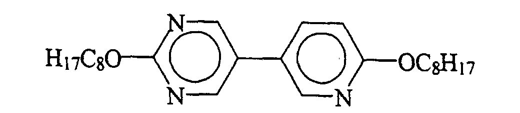

5-(6-Octyloxypyridin-3-yl)-2-octyloxypyrimidin

Zu einer auf 50°C erwärmten Suspension von 1,38 g NaH (80%) in 10 ml DMF wird eine Lösung von 7,2 g (55,3 mmol) 1-Octanol in 20 ml DMF zugetropft. Die Reaktionslösung wird 90 Minuten auf 80°C erhitzt. Dann tropft man eine Lösung von 10 g (42 mmol) 2,5-Dibrompyridin in 35 ml DMF bei dieser Temperatur zu. Das Reaktionsgemisch wird weitere 6 Stunden bei 80°C gerührt. Man gießt die Lösung auf 200 ml Eiswasser, extrahiert 4mal mit Dichiormethan, trocknet die vereinigten organischen Phasen und destilliert das Solvens ab. Das Produkt 2-Octyloxy-5-bompyridin wird an Kieselgel mit Heptan/Ethylacetat = 7/1 chromatographisch gereinigt.

Analog der voranstehenden Vorschrift werden 5,95 g 2,5-Dibrompyrimidin mit 7.9 g 1-Octanol zu 2-Octyloxy-5-brompyrimidin umgesetzt. Das Produkt wird an Kieselgel chromatographisch gereinigt.

2.72 g (10 mmol) 2-Octyloxy-5-bromppyridin werden in 60 ml absolutem Diethylether gelöst und unter Schutzgas auf -75°C abgekühlt. Man versetzt mit 16 mmol 1,6 M-Butyllithiumlösung in Hexan und rührt 15 Minuten bei -75°C nach Anschließend tropft man 4,5 g Triisopropylborat in 50 ml absolutem Dietnytether zu. Die Kühlung wird entfernt. Nachdem der Ansatz sich auf Raumtemperatur erwärmt hat, versetzt man mit 50 ml 10% HCI und rührt 30 Minuten bei Raumtemperatur nach. Man trennt die Phasen und wäscht die organische Phase 2mal mit gesättigter NaCl-Lösung. Man destilliert das Solvens im Vakuum ab und kristallisiert den Rückstand aus Acetonnitril um. Man erhält 6-Octyloxypyridin-3-boronsäure.

In ein Gemisch aus 39 ml Benzol, 26 ml Ethanol und 13 ml Wasser trägt man unter Rühren 1,36 g (5,42 mmol) 6-Octyloxypyridin-3-boronsäure und 1,30 g (4,52 mmol) 2-Octyloxy-5-brompyrimidin ein. Unter Rühren fügt man 1,15 g (10,85 mmol) Na₂CO₃ zu sowie 50 mg Tetrakis (triphenylphosphan)palladium(o). Das Reaktionsgemisch wird 5 Stunden zum Rückfluß erhitzt. Es wird vom ungelösten abfiltriert, der Rückstand Ethanol nachgewaschen und das Solvens entfernt. Das Produkt wird chromatographisch an Kieselgel gereinigt.
X 64 S_{C} 67 S_{A} 91 I

### Beispiel 16

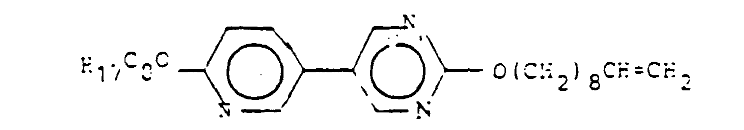

5-(6-Octyloxypyrid-3-yl)-2-(9-decenyloxy)pyrimidin

Analog Beispiel 15 aus 2-(9-Deanyloxy)-5-brompyridin und 6-Octyloxypyridin-3-boronsäure.
X 60 S_{C} 65 S_{A} 80 I N.N.

### Beispiel 17

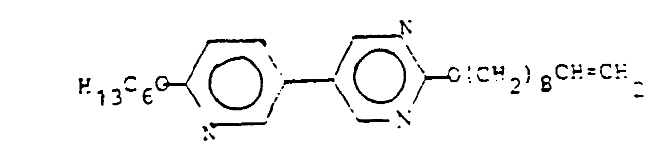

5-(6-Hexyloxypyrid-3-yl)-2-(9-decenyloxy)pyrimidin

Analog Beispiel 15 aus 2-(9-Deanyloxy)-5-brompyrlmidin und 6-Hexyloxypyridin-3-boronsäure
X 53 S_{A} 81 I N.N.

### Anwendungsbeispiel 1

### Ferroelektrische Flüssigkristallmischung

Es wird eine ferroelektrische Mischung bestehend aus den Komponenten

| | |
|---|---|
| 5-Octyloxy-2-(4-hexyloxy-phenyl)-pyrimidin | 22,8 Mol-% |
| 5-Octyloxy-2-(4-butyloxy-phenyl)-pyrimidin | 24 Mol-% |
| 5-Octyloxy-2-(4-decyloxy-phenyl)-pyrimidin | 19,2 Mol-% |
| 5-Octyloxy-2-(4-octyloxy-phenyl)-pyrimidin | 10,5 Mol-% |
| trans-4-Pentyl-cyclohexancarbonsäure-(4'-(5-decylpyrimidin-2-yl)-phenylester | 13,5 Mol-% |
| (2S,3S)-3-Butyloxiran-2-methyl-[2-(6-octyloxypyrid-3-yl)pyrimidin-5-yl)-ether | 10 Mol-% |

hergestellt. Diese zeigt folgende flüssigkristalline Phasenbereiche:
S_{C}^{*}81 S_{A}^{*}98 N^{*}103 I
und weist bei einer Temperatur von 20°C eine spontane Polarisation von
6,8 nC/cm und bei einer Feldstärke von 10 V/µm eine Schaltzeit von 250 µs auf.

### Vergleichsbeispiel III

Im Vergleich dazu weist die in DE 38 31 226.3 beschriebene flüssigkristalline Mischung, die sich von der obengenannten Mischung nur dadurch unterscheidet, daß sie keine (2S,3S)-3-Butyloxiran-2-methyl-[2-(6-octyloxypyrid-3-yl)pyrimidin-5-yl]-ether enthält, folgende Phasenbereiche auf:
S_{C}^{*}84 S_{A}^{*}93N^{*}105 I

Die Zugabe der erfindungsgemäßen Verbindung führt zu einer Erhöhung des S_{C}^{*}-Phasenbereiches.
Dieses Beispiel belegt, daß die erfindungsgemäßen Verbindungen für den Einsatz in ferroelektrischen Mischungen geeignet sind. u.a. weil sie als Dotierstoffe zu kurzen Schaltzeiten führen. Außerdem verfügen diese Komponenten über eine gute Mischbarkeit mit Phenylpyrimidinen.

### Anwendungsbeispiel 2

### Es wird eine binäre Mischung bestehend aus

A 45 Mol-% 2-(4-Octyloxyphenyl)-pyrimidin-5-yl-heptansäureester
   (Phasenfolge: X 69 S_{C}65 N 89 I) und
B 55 Mol-% der erfindungsgemäßen Verbindung:
   2-(Octyloxypyridin-5-yl)-octyloxypyrimidin
hergestellt. Diese zeigt die Phasen:
X 62 S_{C} 71 S_{A} 85 N 88 I
Die Zugabe der erfindungsgemäßen Verbindung (B) führt zu einer verbesserten Mischung, die einen niedrigeren Schmelzpunkt aufweist.

Außerdem weist diese Mischung im Gegensatz zur Ausgangskomponente (A) ein S_{A}-Phase auf, die bei ferroelektrischen Mischungen für eine gute Orientierung der Mischung bei dem Füllen des Displays notwendig ist. Mit den neuen erfindungsgemäßen Verbindungen kann überraschenderweise eine S_{A}-Phase in Mischungen induziert werden.

### Anwendungsbeispiel 3

Es wird eine binäre Mischung hergestellt aus:
C 62,5 Mol-% 2-(4-Octyloxyphenyl)-pyrimidin-5-yl-heptansäureester und (Phasenfolge: X 69 S_{C} 65 N 89 I)
D 37,5 Mol-% der erfindungsgemäßen Verbindung:
   2-(Octyloxypyridin-5-yl)-5-octyloxypyrimidin.
Diese zeigt die Phasen:
   X 54 S_{C} 70 N 91 I

Die Zugabe der erfindungsgemäßen Verbindung (D) führt zu einer verbesserten Mischung, die einen wesentlich niedrigeren Schmelzpunkt autweist.

Die erfindungsgemäßen Verbindungen sind besonders geeignet, in Mischung mit Phenylpyrimidinen den Schmelzpunkt abzusenken.

### Anwendungsbeispiel 4

Eine Flüssigkristallmischung bestehend aus den Komponenten

| | |
|---|---|
| 5-Octyl-2-(4 hexyloxy-phenyl)-pyrimidin | 20,9 Mol-% |
| 5-Octyl-2-(4 decyloxy-phenyl)-pyrimidin | 13,9 Mol-% |
| 5-Decyl-2-(4 hexyloxy-phenyl)-pyrimidin | 13,6 Mol-% |
| 5-Octyl-2-(4'-(7"-cyclopropylheptyloxy)phenyl)-pyrimidin | 11,4 Mol-% |
| 5-Octyl-2-(4-(6''cyclopropyl)-hexylcarbonyloxy-phenyl)-pyrimidin | 14,0 Mol-% |
| 5-(8'''-Cydopropyloctyloxy)-2-(4"-transpentyl-cyclohexyl-4'phenyl)-pyrimidin | 16,2 Mol-% |
| 2-(8-Hexyloxypyrid-3-yl)-5-(8-cyclopropyloctyloxy)-Pyrimidin | 10,0 Mol-% |

zeigt folgende flüssigkristalline Phasenbereiche:
X - 10.6 S_{C} 62 S_{A} 77 N 81 I

Im Vergleich dazu weist die flüssigkristalline Mischung, die sich von der der obengenannten Mischung nur dadurch unterscheidet, daß sie die letzte (enindungsgemäße) Verbindung nicht enthält,
X - 0.5 S_{C} 63 S_{A} 72 N 81 I
Die Zugabe der erfindungsgemäßen Verbindung führt zu einer Herabsenkung des Schmelzpunktes. Die Verbreiterung der S_{A}-Phase bewirkt eine bessere Orientierbarkeit der Flüssigkristallmischung in einem elektrooptischen Anzeigeelement.

### Anwendungsbeispiel 5

Eine Flüssigkristallmischung bestehend aus den Komponenten

| | |
|---|---|
| 5-Octyl-2-(4 hexyloxy-phenyl)-pyrimidun | 14,6 Mol-% |
| 5-Octyl-2-(4 decyloxy-phenyl)-pyrimidin | 9,7 Mol-% |
| 5-Decyl-2-(4 hexyloxy-phenyl)-pyrimidin | 9,4 Mol-% |
| 5-Octyl-2-(4'-/7"-cyclopropylheptyloxy)phenyl)-pyrimidin | 7,9 Mol-% |
| 5-Octyl-2-(4-(6"cyclopropyl)-hexylcarbonyloxyphenyl)-pyrimidin | 9,8 Mol-% |
| 5-(8'''-Cyclopropyloctyloxy)-2-(4"-transpentyl-cyclohexyl-4'phenyl)-pyrimidin | 11,3 Mol-% |
| trans-4-Pentyl-cyclohexancarbonsäure4'-(8"-Cyclopropyloctyl)-pyrimidin-2-ylphenylester | 7,0 Mol-% |
| 5-(5"cyclopropylpentyloxy-2-(4'-hexyloxyphenyl)-pyrimidin | 10,4 Mol-% |
| 2-(4'-Hexylphenyl)-5-(4"-(4'''cyclopropylbutyloxy-phenyl)-pyrimidin | 9,9 Mol-% |
| 5-(6-Octylpyridin-3-yl)-2-(9-Decenyloxy)pyrimidin | 10,0 Mol-% |

zeigt folgende flüssigkristalline Phasenbereiche:
X - 7 S_{C} 69 S_{A} 84 N 93 I

### Anwendungsbeispiel 6

Eine Flüssigkristallmischung bestehend aus den Komponenten

| | |
|---|---|
| 5-Octyl-2-(4 hexyloxy-phenyl)-pyrimidin | 14,6 Mol-% |
| 5-Octyl-2-(4 decyloxy-phenyl)-pyrimidin | 9,7 Mol-% |
| 5-Decyl-2-(4 hexyloxy-phenyl)-pyrimidin | 9,4 Mol-% |
| 5-Octyl-2-(4'-(7"-cyctopropylheptyloxy)phenyl)-pyridin | 7,9 Mol-% |
| 5-Octyl-2-(4-(6"cyclopropyl)-hexylcarbonyloxyphenyl)-pyrimidin | 9,8 Mol-% |
| 5-(8'''-Cyclopropyloctyloxy)-2-(4''-transpentyl-cyclohexyl-4'phenyl)-pyrimidin | 11,3 Mol-% |
| trans-4-Pentyl-cyclohexancarbonsäure4'-(8"-Oyclopropyloctyl)-pyrimidin-2-ylphenylester | 7,0 Mol-% |
| 5-(5"Cyclopropylpentyloxy-2-(4'-hexyloxyphenyl)-pyrimidin | 10,4 Mol-% |
| 2-(4'-Hexylphenyl)-5-(4"-(4'''cyclopropylbutyloxy-phenyl)-pyrimidin | 9,9 Mol-% |
| 5-(6-Octyloxypyridin-3-yl)-2-octyloxypyrimidin | 10,0 Mol-% |

zeigt folgende flüssigkristalline Phasenbereiche:
X - 7 S_{C} 69 S_{A} 83 N 94 I
Eine Flüssigkristallmischung, die sich nur dadurch von den beiden letztgenannten Mischungen unterscheidet, daß sie die letzte erfindungsgemäße Verbindung nicht enthält, zeigt die Phasenbereiche
X - 4 S_{C} 72 S_{A} 80 N 96 I
Die Zugabe der erfindungsgemäßen Verbindung führt neben einer Absenkung des Schmelzpunktes zu einer vorteilhaften Verbreiterung des S_{A}-Phasenbereiches.

### Anwendungsbeispiel 7

Eine Mischung, die aus den Komponenten

| | |
|---|---|
| 5-Heptyl-2-(4-pentyloxy-phenyl)-pyrimidin | 13,8 Mol-% |
| 5-Heptyl-2-(4-heptyloxy-phenyl)-pyrimidin | 9,2 Mol-% |
| 5-Heptyl-2-(4- butyloxy-phenyl)-pyrimidin | 14,8 Mol-% |
| 5-(6"-Cyclopropyl)hexyloxy-2-(4'octyloxyphenyl)-pyrimidin | 14,0 Mol-% |
| trans-4-Pentyl-cyclohexylmethyloxy-5-(8"-Cyclopropyloctyloxy)-pyrimidin-2-yl-phenyl | 6,4 Mol-% |
| trans-4-Pentyl-cyclohexancarbonsäure-5-(8"-Gyclopropyloctyl)-pyrimidin-2-yl-phenylester | 11,1 Mol-% |
| 5-Octyl-2-(4-(6"cyclopropyl)-hexylcarbonyloxy-phenyl)-pyrimidin | 20,7 Mol-% |
| 2-(6-Hexyloxypyrid-3-yl)-5-hexyloxypyrimidin | 10,0 Mol-% |

besteht, zeigt folgende flüssigkristalline Phasenbereiche:
X 2.1 S_{C} 54 S_{A} 73 N 91 I
Im Vergleich dazu weist die flüssigkristalline Mischung, die sich von der obengenannten Mischung nur dadurch unterscheidet, daß sie keine erfindungsgemäße Verbindung aufweist, folgende Phasenbereiche auf:
X 3.4 S_{C} 61 N 92 I
Der Vergleich zeigt, daß schon bei geringem Zusatz der erfindungsgemäßen Verbindung in flüssigkristallinen Mischungen eine S_{A}-Phase erzeugt werden kann bei gleichzeitiger Absenkung des Schmelzpunktes.

## Patentansprüche

1. Pyridylpyrimidin-Verbindungen der allgemeinen Formel I 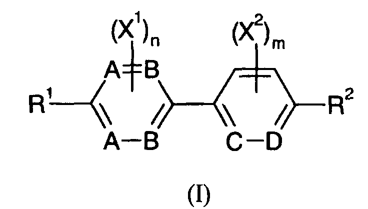 in der bedeuten:
A gleich N und B gleich CH oder A gleich CH und B gleich N
C gleich N und D gleich CH oder C gleich CH und D gleich N
R¹ und R² gleich oder verschieden lineares oder verzweigtes Alkyl mit 1 bis 16 C-Atomen, Alkyloxy oder Alkyldimethylsilylalkyl(oxy) der Formel R⁷-Si(CH₃)₂-G oder einer der nachfolgenden Reste 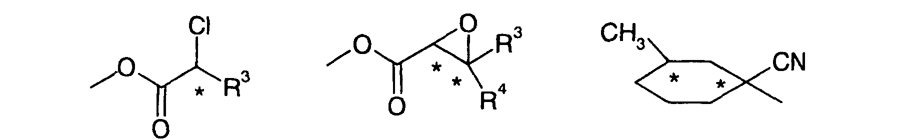 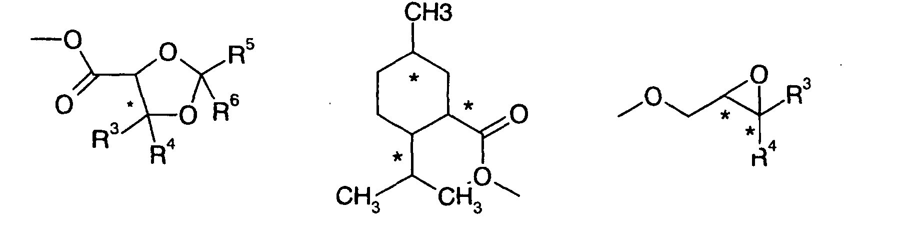 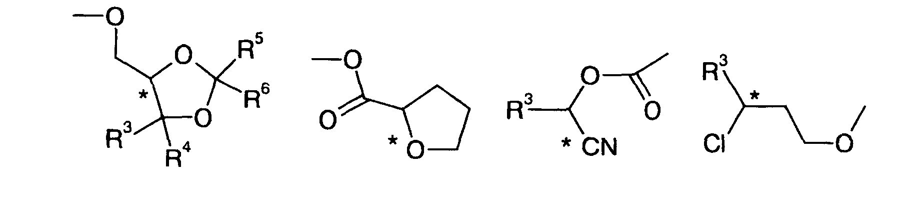 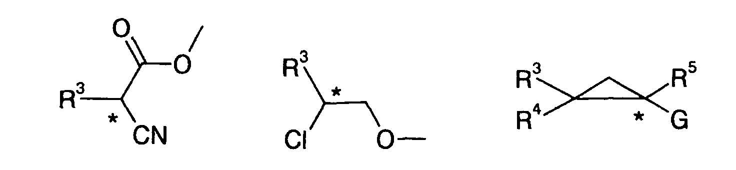 oder trans-Alkylcyclohexylcarbonyloxy,
X¹, X² gleich F, Cl, CN,
n, m gleich oder verschieden 0, 1 oder 2,
R³, R⁴, R⁵, R⁶ H, geradkettiges oder verzweigtes Alkyl mit 1 bis 16 C-Atomen oder Alkenyl mit 2 bis 16 C-Atomen, bei denen auch eine nicht der Verknüpfungsstelle benachbarte -CH₂-Gruppe durch -O-, -CO-O- oder -O-CO- ersetzt sein können, oder
R³ und R⁴ bzw. R⁵ und R⁶ zusammen cyclisches Alkyl mit 3 bis 8 C-Atomen, R⁷ geradkettiges oder verzweigtes Alkyl mit 1 bis 16 C-Atomen oder Alkenyl mit 2 bis 16 C-Atomen, bei denen auch eine oder zwei nicht benachbarte -CH₂-Gruppen durch -O-, -CO-O- oder -O-CO- ersetzt sein können, mit der Maßgabe, daß Silizium nur an ein C gebunden ist, das H und/oder C als Nachbaratome hat, oder cyclisches Alkyl mit 3 bis 8 C-Atomen,
G geradkettiges oder verzweigtes Alkylen mit 1 bis 16 C-Atomen oder Alkenylen mit 2 bis 16 C-Atomen, bei denen eine oder zwei nicht benachbarte -CH₂-Gruppen durch -O-, -S-, -O-CO-, -CO-O-, -S-CO- oder -CO-S- ersetzt sein können.

2. Pyridylpyrimidin gemäß Anspruch 1, **dadurch gekennzeichnet, daß** in der allgemeinen Formel (I) R¹ und R² gleich oder verschieden lineares oder verzweigtes Alkyl mit 1 bis 16 C-Atomen, Alkyloxy oder Alkyldimethylsilylalkyl(oxy) der Formel R⁷-Si(CH₃)₂-G, trans-Alkylcyclohexylcarbonyloxy oder 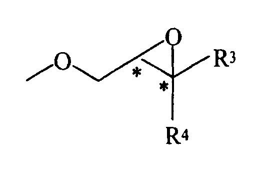 bedeuten.

3. Pyridylpyrimidin gemäß Anspruch 1, **dadurch gekennzeichnet, daß** es der allgemeinen Formel (II) oder (III) entspricht, 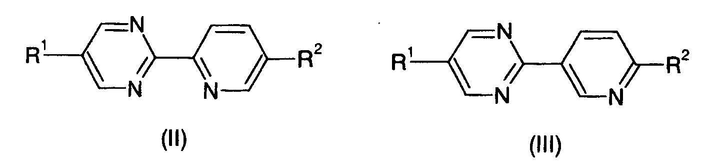 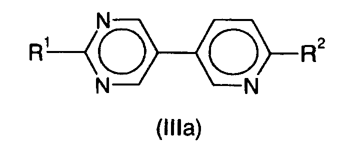 wobei R¹ und R² die in Anspruch 1 genannte Bedeutung haben.

4. Verfahren zur Herstellung der Pyridylpyrimidin-Verbindungen der Formel (I) gemäß Anspruch 1, durch Kondensation eines reaktionsfähigen Pyridinderivates mit einer bifunktionellen elektrophilen Verbindung unter Bildung eines Pyrimidinsystems.

5. Verwendung der Pyridylpyrimidin-Verbindungen der allgemeinen Formel (I) nach Anspruch 1 als Komponente in Flüssigkristallmischungen.

6. Verwendung nach Anspruch 5, **dadurch gekennzeichnet, daß** die Flüssigkristallmischung nematisch ist.

7. Verwendung nach Anspruch 5, **dadurch gekennzeichnet, daß** die Flüssigkristallmischung ferroelektrisch ist.

8. Flüssigkristalline Mischung bestehend aus mindestens zwei Komponenten, **dadurch gekennzeichnet, daß** sie mindestens eine Pyridylpyrimidin-Verbindung der allgemeinen Formel (I) nach Anspruch 1 enthält.

9. Elekrooptische Schalt- und Anzeigevorrichtung, enthaltend Trägerplatten, Orientierungsschichten, Elektroden sowie ein flüssigkristallines Medium, **dadurch gekennzeichnet, daß** das flüssigkristalline Medium eine Mischung gemäß Anspruch 8 ist.

## Claims

1. A pyridylpyrimidine compound of the formula I 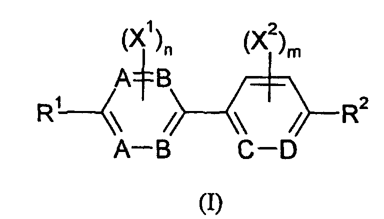 in which
A is N and B is CH or A is CH and B is N,
C is N and D is CH or C is CH and D is N,
R¹ and R², identical or different, are straight-chain or branched alkyl having 1 to 16 carbon atoms, alkyoxy, alkyl dimethylsilylalkyl(oxy) of the formula R⁷-Si(CH₃)₂-G or one of the following radicals 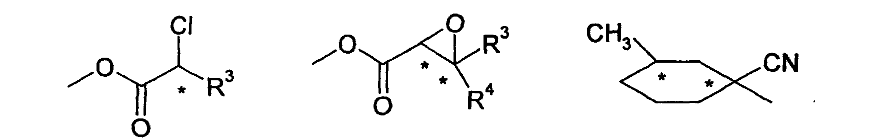 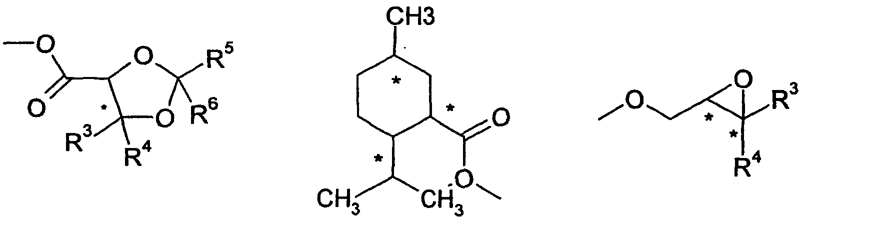 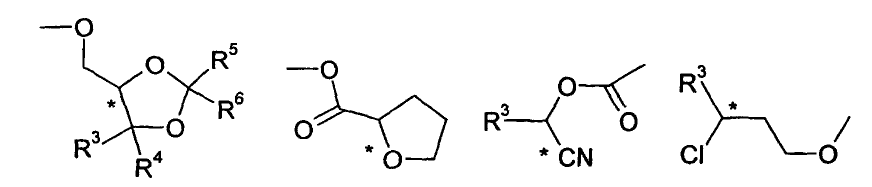 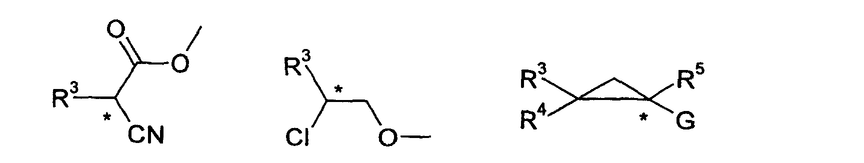 or trans-alkylcyclohexylcarbonyloxy,
X¹, X² are F, Cl, CN,
n, m, identical or different, are zero, 1 or 2,
R³, R⁴, R⁵, R⁶ are H, straight-chain or branched alkyl having 1 to 16 carbon atoms or alkenyl with 2 to 16 carbon atoms, in which a -CH₂- group not adjacent to the linkage point can also be replaced by -O-, -CO-O- or -O-CO- or
R³ and R⁴ or R⁵ and R⁶ together are cyclic alkyl having 3 to 8 carbon atoms,
R⁷ is straight-chain or branched alkyl having 1 to 16 carbon atoms or alkenyl having 2 to 16 carbon atoms, in which one or two non-adjacent -CH₂- groups can also be replaced by -O-, -CO-O- or -O-CO-, with the proviso that silicon is only bound to a carbon which has hydrogen and/or carbon as neighboring atoms, or is cyclic alkyl having 3 to 8 carbon atoms,
G is straight-chain or branched alkylene having 1 to 16 carbon atoms or alkenylene having 2 to 16 carbon atoms, in which one or two non-adjacent -CH₂- groups can also be replaced by -O-, -S-, -O-CO-, -CO-O-, -S-CO- or -CO-S-.

2. A pyridylpyrimidine as claimed in claim 1, wherein in the formula (I) R¹ and R², identical or different, are linear or branched alkyl having 1 to 16 carbon atoms, alkyloxy or alkyldimethylsilylalkyl(oxy) of the formula R⁷-Si(CH₃)₂-G, transalkylcyclohexylcarbonyloxy or

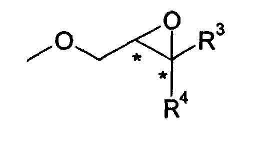

3. A pyridylpyrimidine as claimed in claim 1, having the formula (II) or (III), 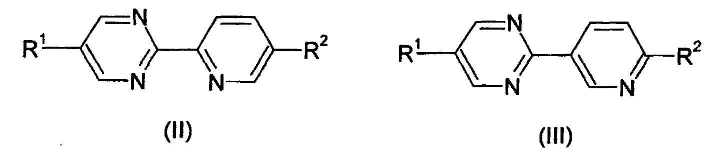 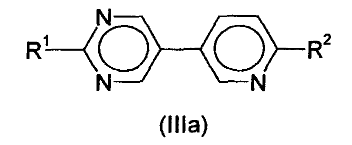 R¹ and R² having the meaning given in claim 1.

4. A process for the preparation of a pyridylpyrimidine compound of the formula (I) as claimed in claim 1, by condensation of a reactive pyridine derivative with a bifunctional electrophilic compound with the formation of a pyrimidine system.

5. The use of a pyridylpyrimidine compound of the formula (I) as claimed in claim 1 as a component in liquid crystal mixtures.

6. The use as claimed in claim 5, wherein the liquid crystal mixture is nematic.

7. The use as claimed in claim 5, wherein the liquid crystal mixture is ferroelectric.

8. A liquid crystalline mixture composed of at least two components, wherein the mixture contains at least one pyridylpyrimidine compound of the formula (I) as claimed in claim 1.

9. An electrooptical switching and display device, which contains baseplates, orientation layers, electrodes and a liquid-crystalline medium, wherein the liquid-crystalline medium is a mixture as claimed in claim 8.

## Revendications

1. Composés de pyridylpyrimidine de formule générale I 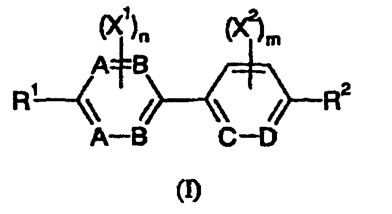 dans laquelle
A représente un atome de N et B représente un groupe CH ou A représente un groupe CH et B représente un atome de N
C représente un atome de N et D représente un groupe CH ou C représente un groupe CH et D représente un atome de N
R¹ et R² sont identiques ou différents et représentent des groupes alkyle présentant 1 à 16 atomes de carbone linéaire ou ramifié, alkyloxy ou alkyldiméthylsilylalkyl(oxy) de formule R⁷-Si(CH₃)₂-G ou un des restes suivants 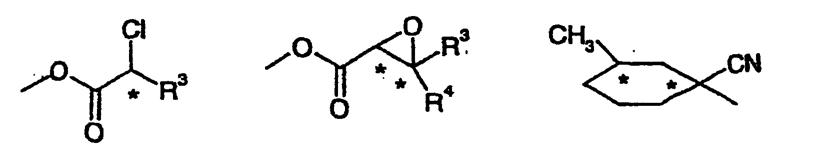 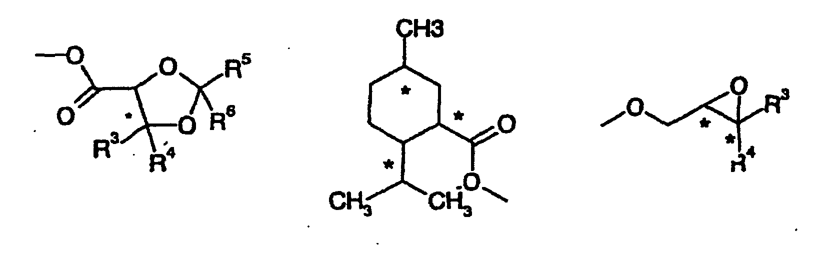 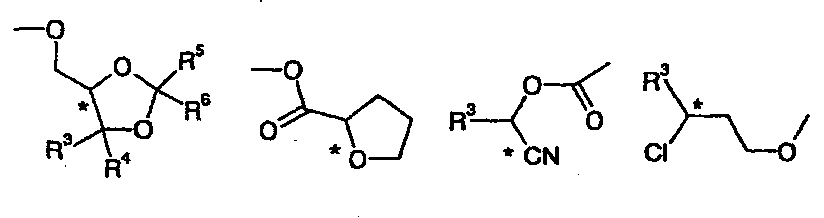 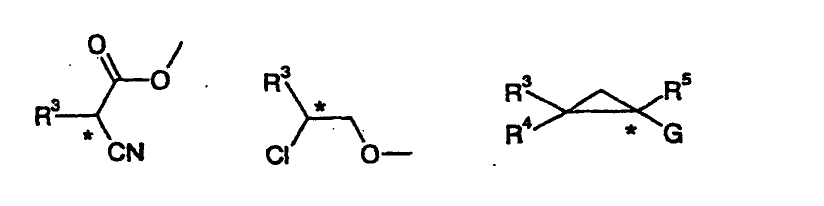 ou un groupe trans-alkylcyclohexylcarbonyloxy,
X¹, X² représentent des groupes F, Cl, CN,
n, m sont identiques ou différents et valent 0, 1 ou 2, b
R³, R⁴, R⁵, R⁶ représentent un atome de H, un groupe alcényle présentant 2 à 16 atomes de carbone ou alkyle présentant 1 à 16 atomes de carbone linéaire ou ramifié, dans lesquels un groupe -CH₂- non voisin à l'endroit de liaison peut également être remplacé par des groupes -O-, -CO-O- ou -O-CO-, ou
R³ et R⁴, respectivement R⁵ et R⁶ conjointement peuvent représenter des groupes alkyle cyclique présentant 3 à 8 atomes de carbone, R⁷ représente un groupe alcényle présentant 2 à 16 atomes de carbone ou alkyle linéaire ou ramifié présentant 1 à 16 atomes de carbone, dans lesquels un ou deux groupes -CH₂- non voisins peuvent également être remplacés par des groupes -O-, -CO-O- ou -O-CO-, à condition que le silicium soit lié uniquement à un atome de carbone qui a pour atomes voisins des atomes d'hydrogène et/ou de carbone, ou un groupe alkyle cyclique présentant 3 à 8 atomes de carbone,
G représente des groupes alcénylène présentant 2 à 16 atomes de carbone ou alkylène présentant 1 à 16 atomes de carbone, linéaire ou ramifié, dans lesquels un ou deux groupes -CH₂- non adjacents peuvent être remplacés par des groupes -O-, -S-, -O-CO-, -CO-O-, -S-CO- ou -CO-S-.

2. Pyrimidylpyrimidine selon la revendication 1, **caractérisée en ce que** dans la formule générale (I) R¹ et R², identiques ou différents, représentent des groupes alkyle présentant 1 à 16 atomes de carbone linéaire ou ramifié, alkyloxy ou alkyldiméthylsilylalkyl(oxy) de formule R⁷-Si(CH₃)₂-G, transalkylcyclohexylcarbonyloxy ou

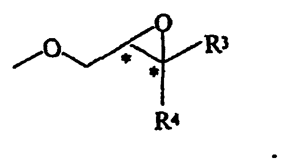

3. Pyridylpyrimidine selon la revendication 1, **caractérisée en ce qu'**elle répond à la formule générale (II) ou (III), 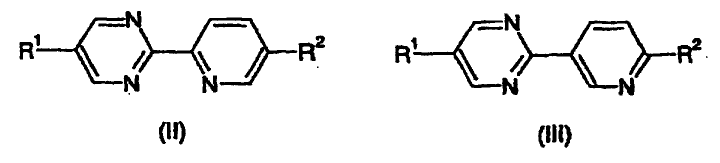 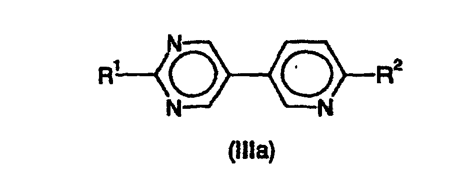 où R¹ et R² possèdent la signification donnée à la revendication 1.

4. Procédé pour la préparation des composés de pyridylpyrimidine de formule (I) selon la revendication 1, par condensation d'un dérivé réactif de la pyridine sur un composé bifonctionnel électrophile en formant un système pyridimidine.

5. Utilisation des composés de pyridylpyrimidine de formule générale (I) selon la revendication 1 en tant que composant dans des mélanges de cristaux liquides.

6. Utilisation selon la revendication 5, **caractérisée en ce que** le mélange de cristaux liquides est nématique.

7. Utilisation selon la revendication 5, **caractérisée en ce que** le mélange de cristaux liquides est ferroélectrique.

8. Mélange de cristaux liquides constitué par au moins deux composants, **caractérisé en ce qu'**il renferme au moins un composé de pyridylpyrimidine de formule générale (I) selon la revendication 1.

9. Dispositif d'affichage et de commutation électro-optique constitué par des plaques supports, des couches d'orientation, des électrodes, ainsi qu'un milieu de cristal liquide, **caractérisé en ce que** le milieu de cristal liquide est un mélange selon la revendication 8.
